# EUROPEAN PATENT APPLICATION

(11) **EP 2 562 157 A1**
(43) Date of publication of application: **27.02.2013**
(21) Application number: 11771588.8
(22) Date of filing: 21.04.2011
(51) Int. Cl.: C07C 333/20, C07C 333/22, C07C 333/24, C07D 417/02, C07D 417/14, C07D 213/40, C07D 213/75, C07D 239/26, C07D 241/12, C07D 307/52, C07D 263/32, C07D 277/28, C07D 271/10, C07D 215/12, C07D 405/04, C07D 498/04, A61K 31/27, A61K 31/541, A61K 31/341, A61K 31/44

(54) **HETEROARYL (ALKYL) DITHIOCARBAMATE COMPOUNDS, PREPARATION METHODS AND USES THEREOF**

(30) Priority: 21.04.2010 CN 201010152113
(71) Applicant: Peking University, Beijing 100191 (CN)
(72) Inventor: LI, Runtao, Beijing 100191 (CN); GE, Zemei, Beijing 100191 (CN); CUI, Jingrong, Beijing 100191 (CN); SUN, Xingyi, Beijing 100191 (CN); WANG, Zhongqing, Beijing 100191 (CN); YAN, Xu, Beijing 100191 (CN)
(74) Representative: Müller Schupfner & Partner
(86) International application number: PCT/CN2011/073118
(87) International publication number: WO 2011/131135

(57) **Abstract**

Heteroaryl (alkyl) dithiocarbamate compounds represented by general formula (I) or their pharmaceutically acceptable salts, their preparing methods, and their uses for preparing antitumor medicines are disclosed, wherein each said substituent is defined as in the description. The compounds are new tyrosine kinase inhibitors useful as an anti-tumor agents, preferably useful in the preparation of medicines for treating breast cancer, liver cancer, non-small cell lung cancer, gastric cancer, colon cancer, leukaemia or nasal cancer.

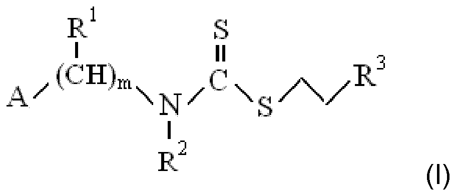

## Description

### FIELD OF THE INVENTION

The present invention relates to a novel type of heteroaryl (alkyl) dithiocarbamate compounds, preparation methods and uses thereof, and the compounds of the present invention are a novel type of tyrosine kinase inhibitors and may be used as anti-tumor drugs.

### BACKGROUND OF THE INVENTION

The epidermal growth factor receptor (ErbB) family belongs to type I family of tyrosine kinase, and has four members: ErbB-1 (EGFR), ErbB-2 (HER-2), ErbB-3 and ErbB-4. It is demonstrated by researches that, in tumor cells such as bladder cancer, breast cancer, colorectal cancer, lung cancer and the like, EGFR and HER-2 are both over-expressed, and solid tumor is closely relevant to their over-expression. Therefore, in recent years, the research of anti-tumor drugs using EGFR and HER-2 as targets has attracted great attention.

So far, the known EGFR and HER-2 inhibitors mainly include five structural types: 4-aminoquinazolines, 4-amino-3-cynoquinolines, benzalmalononitriles, salicylamides and pyrrolotriazines. Among these five structural types, 4-aminoquinazolines and 4-amino-3-cynoquinolines are mostly studied, such as the compounds disclosed in Chinese patent application CN200610023526.7 and International Publications W02008/0033748 and W02008/0033749. A variety of compounds with great anti-tumor activity have been found, and some of them have been used clinically.

Dithiocarbamate compounds are another type of anti-tumor compounds, while W02007/050963A1 has disclosed a series of dithiocarbamate compounds: 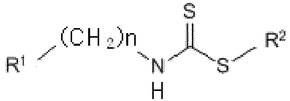 wherein R¹ is cycloalkyl, aryl or benzopyrrolyl, benzofuryl or benzothienyl; the compounds may be used as anti-tumor drugs; Runtao Li et al. have disclosed a variety of such compounds in Chinese patent applications CN01118399.3, CN200410054686.9 and CN200910143757.5, in which R¹ and R² are of different types.

According to the researching results of dithiocarbamate compounds in the prior art and the researching development of the epidermal growth factor receptor inhibitor (EGFR inhibitor), through a lot of experiments, the present inventors develop a series epidermal growth factor receptor inhibitors with novel structures, great activity, high selectivity and low toxicity.

### Summary of the invention

The objective of the present invention is to provide a series of compounds with novel structures, represented by the general formula (I): 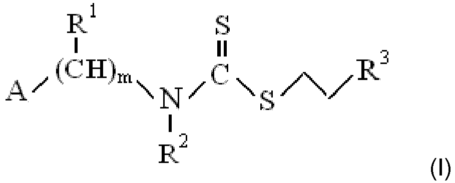 or their pharmaceutically acceptable salts,
wherein:
A is substituted or unsubstituted, five- or six-membered heterocyclic group having one or more heteroatoms selected from nitrogen, oxygen and sulfur, which may be substituted with one or more substituents selected from halogen, C₁₋₄alkyl, C₁₋₄alkoxy, phenyl, phenoxy, furyl, morpholinyl/morpholino, C₁₋₆alkylamido and C₁₋₄alkoxycarbonyl; when the heterocyclic group is pyridyl, it may be fused with benzene or heterocyclic group, such fused benzene or heterocyclic group is unsubstituted or substituted with C₁₋₄alkyl;
R¹ is H, phenyl, or C₁₋₄alkyl;
R² is H or C₁₋₄alkyl;
R³ is cyano, borono, C₁₋₄alkoxycarbonyl, aminosulfonyl, benzylsulfinyl, or C₁₋₄alkylsulfinyl; or R² and R³ link together to form saturated or unsaturated, five- or six-membered heterocyclic group containing nitrogen and sulfur atoms, and the heterocyclic group is unsubstituted or substituted with one or more hydroxy or C₁₋₄alkyl; and
m is an integer between 0 and 3.

In the compounds of the present invention, the group A is substituted or unsubstituted heterocyclic group; preferably, group A is substituted or unsubstituted pyridyl, pyrimidinyl, pyrazinyl, furyl, oxazolyl, pyrazolyl, thiazolyl or oxadiazolyl; wherein the heterocyclic group may be substituted with one or more substituents selected from halogen, C₁₋₄alkyl, C₁₋₄alkoxy, phenyl, phenoxy, furyl, morpholinyl/morpholino, C₁₋₆alkylamido and C₁₋₄alkoxycarbonyl; or, when the heterocyclic group is pyridyl, the pyridyl may be fused with benzene or heterocyclic group, and the fused benzene or heterocyclic group is unsubstituted or substituted with C₁₋₄alkyl.

In the above definition of the compounds of the present invention, the term "C₁₋₄alkyl" used herein refers to linear or branched, saturated alkyl radicals having 1 to 4 carbon atoms, preferably linear or branched alkyl radicals having 1 to 3 carbon atoms, such as methyl, ethyl, propyl, isopropyl or the like; more preferably methyl and ethyl, most preferably methyl.

In the above definition of the compounds of the present invention, the term "C₁₋₄alkoxy" used herein refers to linear or branched, saturated alkoxy radicals having 1 to 4 carbon atoms, such as methoxy, ethoxy, propoxy or isopropoxy; preferably methoxy or ethoxy; more preferably methoxy.

In the above definition of the compounds of the present invention, the term "C₁₋₄alkoxycarbonyl" used herein refers to linear or branched, saturated alkoxycarbonyl radicals having 1 to 4 carbon atoms, such as methoxycarbonyl, ethoxycarbonyl, propoxycarbonyl or isopropoxycarbonyl; preferably methoxycarbonyl or ethoxycarbonyl; more preferably methoxycarbonyl.

In the above definition of the compounds of the present invention, the term "C₁₋₆alkylamido" used herein refers to linear or branched, saturated alkylamido radicals having 1 to 6 carbon atoms, such as methylamido, ethylamido, propylamido, or isomers of butylamido, pentylamido, hexylamido; preferably branched alkylamido radicals having 4 to 6 carbon atoms, more preferably isomers of pentylamido, most preferably isovaleramido or pivaloylamido.

In the above definition of the compounds of the present invention, the term "halogen" used herein refers to fluorine, chlorine, bromine and iodine atoms, preferably fluorine, chlorine or bromine atoms; more preferably bromine atoms.

In the compounds according to the present invention, the preferred group A is substituted or unsubstituted pyridyl.

In the compounds according to the present invention, the preferred R³ group is cyano.

In the preferred compounds according to the present invention, group A is substituted or unsubstituted pyridyl, and R³ is cyano.

The pharmaceutically acceptable salts of the compounds of the general formula (I) according to the present invention refer to acid addition salt of the compounds of the invention, including inorganic or organic acid addition salts, wherein the inorganic acid is such as sulfuric acid, hydrochloric acid, sulfurous acid, boric acid, phosphoric acid, sulfonic acid, hydrobromic acid, hydrofluoric acid and the like; the organic acid is such as acetic acid, valeric acid, maleic acid, fumaric acid, oxalic acid, oleic acid, lactic acid, palmitic acid, lauric acid, stearic acid, citric acid, succinic acid, tartaric acid, benzoic acid, methanesulfonic acid, toluenesulfonic acid, gluconic acid, lactobionic acid, laurylsulfonic acid and the like; among said salts, it may contains alkali metal cations or alkaline earth metal cations, such as lithium, sodium, potassium, calcium, magnesium and the like, and various organic ammonium cations, such as the ammonium cation formed by tetramethyl ammonium, tetraethyl ammonium, methylamine, dimethylamine, ethylamine and the like.

In the above definition of the compounds of the present invention, the term "substituted or unsubstituted" is used, wherein the term "substituted" refers to groups being mono-substituted or poly-substituted with substituents at any substitutable position.

The above-described compounds of the present invention are epidermal growth factor receptor inhibitors (EGFR inhibitor), can be used for the treatment of tumor, and are particularly suitable for the treatment of the diseases mediated by tyrosine kinase, such as breast cancer, liver cancer, non-small cell lung cancer, gastric cancer, colon cancer, leukaemia, nasal cancer and the like.

The preferred compounds of the present invention are:
3-(furan-2-ylmethyl)-4-hydroxy-1,3-thiazinane-2-thione (compound 1);
2-(methoxycarbonyl)ethyl (furan-2-ylmethyl)dithiocarbamate (compound 2);
2-cyanoethyl (furan-2-ylmethyl)dithiocarbamate (compound 3);
3-(furan-2-ylmethyl)-4-hydroxy-4,5-dimethyl-3,4-dihydro-2H-1,3-thiazine-2-thione (compound 4);
2-sulfamoylethyl (furan-2-ylmethyl)dithiocarbamate (compound 5);
2-boronoethyl (furan-2-ylmethyl)dithiocarbamate (compound 6);
2-(methylsulfinyl)ethyl (furan-2-ylmethyl)dithiocarbamate (compound 7);
2-(benzylsulfinyl)ethyl (furan-2-ylmethyl)dithiocarbamate (compound 8);
4-hydroxy-3-(pyridin-3-ylmethyl)-1,3-thiazinane-2-thione (compound 9);
2-(methoxycarbonyl)ethyl (pyridin-3-ylmethyl)dithiocarbamate (compound 10);
2-cyanoethyl (pyridin-3-ylmethyl)dithiocarbamate (compound 11);
4-hydroxy-4,5-dimethyl-3-(pyridin-3-ylmethyl)-3,4-dihydro-2H-1,3-thiazine-2-thione (compound 12);
2-sulfamoylethyl (pyridin-3-ylmethyl)dithiocarbamate (compound 13);
2-boronoethyl (pyridin-3-ylmethyl)dithiocarbamate (compound 14);
2-(methylsulfinyl)ethyl (pyridin-3-ylmethyl)dithiocarbamate (compound 15);
2-(benzylsulfinyl)ethyl (pyridin-3-ylmethyl)dithiocarbamate (compound 16);
2-cyanoethyl [(4-pivalamidopyridin-3-yl)methyl]dithiocarbamate (compound 17);
2-cyanoethyl (quinolin-3-ylmethyl)dithiocarbamate (compound 18);
2-cyanoethyl [2-(pyridin-3-yl)ethyl]dithiocarbamate (compound 19);
2-cyanoethyl [(2-methoxypyridin-3-yl)methyl]dithiocarbamate (compound 20);
2-cyanoethyl [(6-methoxycarbonylpyridin-3-yl)methyl]dithiocarbamate (compound 21);
2-cyanoethyl [(4,6-dimethylpyridin-3-yl)methyl]dithiocarbamate (compound 22);
2-cyanoethyl [(5-methoxypyridin-3-yl)methyl]dithiocarbamate (compound 23);
2-cyanoethyl [(5-bromopyridin-3-yl)methyl]dithiocarbamate (compound 24);
2-cyanoethyl {[6-(furan-2-yl)pyridin-3-yl]methyl}dithiocarbamate (compound 25);
2-cyanoethyl [(4-methyl-3,4,4a,8a-tetrahydro-2*H*-pyrido[3,2-b][1,4]oxazin-7-yl)methyl] dithiocarbamate (compound 26);
2-cyanoethyl [(6-phenoxypyridin-3-yl)methyl]dithiocarbamate (compound 27);
2-cyanoethyl (pyridin-4-ylmethyl)dithiocarbamate (compound 28);
2-cyanoethyl N-methyl-(pyridin-3-ylmethyl)dithiocarbamate (compound 29);
2-cyanoethyl pyridin-3-yldithiocarbamate (compound 30);
2-cyanoethyl [phenyl(pyridin-3-yl)methyl]dithiocarbamate (compound 31);
2-cyanoethyl [1-(pyridin-3-yl)ethyl]dithiocarbamate (compound 32);
2-cyanoethyl [(6-methoxypyridin-3-yl)methyl]dithiocarbamate (compound 33);
2-cyanoethyl [(4-methyl-2-phenyloxazol-5-yl)methyl]dithiocarbamate (compound 34);
2-cyanoethyl [(5-methyl-1-phenyl-1*H*-pyrazol-4-yl)methyl]dithiocarbamate (compound 35);
2-cyanoethyl (pyrimidin-5-ylmethyl)dithiocarbamate (compound 36);
2-cyanoethyl [(1,3-dimethyl-1*H*-pyrazol-5-yl)methyl]dithiocarbamate (compound 37);
2-cyanoethyl (thiazol-2-ylmethyl)dithiocarbamate (compound 38);
2-cyanoethyl (pyrazin-2-ylmethyl)dithiocarbamate (compound 39);
2-cyanoethyl [(5-phenyl-1 ,3,4-oxadiazol-2-yl)methyl]dithiocarbamate (compound 40);
2-cyanoethyl [(2-morpholinopyrimidin-5-yl)methyl]dithiocarbamate (compound 41);
2-cyanoethyl [3-(pyridin-3-yl)propyl]dithiocarbamate (compound 42);
2-cyanoethyl (pyridin-3-ylmethyl)dithiocarbamate hydrochloride (compound 43);
2-cyanoethyl [2-(pyridin-3-yl)ethyl]dithiocarbamate hydrochloride (compound 44);
2-cyanoethyl [(5-phenyl-1 ,3,4-thiadiazol-2-yl)methyl]dithiocarbamate (compound 45);
2-cyanoethyl [(1*H*-pyrrol-2-yl)methyl]dithiocarbamate (compound 46); and
2-(butoxycarbonyl)ethyl (pyridin-3-ylmethyl)dithiocarbamate (compound 47).

Another objective of the present invention is to provide preparation method for the compounds of the general formula (I) or their pharmaceutically acceptable salts as above described, and the compounds of the general formula (I) can be prepared according to the following route: 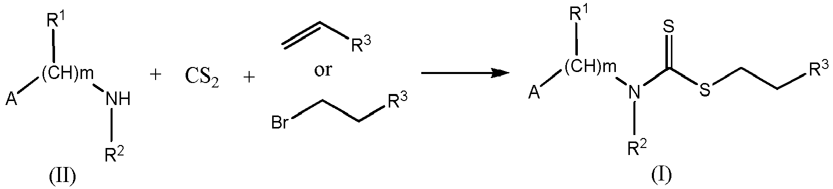 wherein each groups in the general formulae (I) and (II) are defined as above.

If necessary, the pharmaceutically acceptable salts of the compounds can be prepared according to the routine salt-forming process in the chemical field.

The preparation method as described above is a known method for preparing dithiocarbamates in the chemical field. Specifically, the preparation method can be as follows: the compounds of formula (II), in the presence of anhydrous potassium phosphate, in organic solvent such as acetone, are firstly reacted with stoichiometric carbon disulfide, and then reacted with stoichiometric bromoethyl cyanide or alkenes attached with electron withdrawing groups while stirring. After the reaction is finished, the reaction products can be refined according to the routine methods in the chemical field to obtain the desired target compounds.

More specifically, the preparation method may be as follows: 1 equivalence of the compounds of formula (II) are dissolved in acetone, and 1 equivalence of anhydrous potassium phosphate is added; several minutes later, 4 equivalences of carbon disulfide are added, and after 20 minutes, 1 equivalence of bromoethyl cyanide or alkenes attached with electron withdrawing groups are added; the resultant mixture is stirred for several hours at room temperature, the solvent is evaporated; the resultant mixture is diluted with water, and extracted with ethyl acetate; the organic solvent is removed to obtain the crude product, and the crude product is purified by column chromatography to obtain the desired target compound.

The compounds of formula (II) as a raw material, bromoethyl cyanide and alkenes attached with electron withdrawing groups used herein are commercially available, or can be prepared according to the known methods in the art.

Depending on the compounds to be prepared, the specific reaction conditions and assistant reagents may be properly adjusted, and the alternation of the reaction conditions may be easily determined via the routine experiments by those skilled in the art.

For example, when group A in the target compound is furyl and R³ is cyano, the preparation method may be to react furyl(C₁₋₄)alkylamine with carbon disulfide and bromoethyl cyanide, and the preparation method may be carried out in the presence of potassium phosphate, in acetone at room temperature. The furyl therein may be substituted or unsubstituted.

For another example, when R³ in the target compound is borono, the corresponding boric acid ester compound can be prepared firstly. For example, the boric acid ester compound may be prepared according to the following process: at -76°C, using anhydrous THF as solvent, dibromoethane is reacted, under nitrogen atmosphere, with n-BuLi to form 2-bromoethyllithium, which is directly reacted, without further purification, with trimethylborate to obtain 2-bromoethylboric acid ester; then using this compound as a raw material, the target compound is prepared following the preparation of compounds of formula (I) as described above.

For another example, when group A in the compounds of the general formula (I) is substituted or unsubstituted pyridyl, m=0, and R³ is cyano, the compound may be prepared according to the following route: 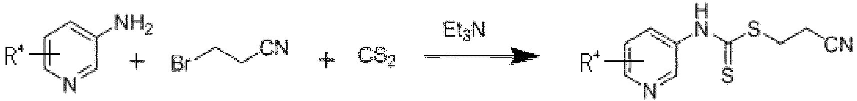 then, as required, the pharmaceutically acceptable salts of the compounds may be prepared according to the routine salt-forming process in the chemical field;
wherein group R⁴ is a substituent on the above-described group A, which is one or more groups selected from halogen, C₁₋₄alkyl, C₁₋₄alkoxy, phenyl, phenoxy, furyl, morpholinyl/morpholino, C₁₋₆alkylamido and C₁₋₄alkoxycarbonyl; or, the pyridyl is fused with benzene or heterocyclic group, preferably benzene or morpholine ring; the fused benzene or heterocyclic group is unsubstituted or substituted with C₁₋₄alkyl, preferably unsubstituted or substituted with methyl.

For example, the detailed preparing method is as follows: 3-aminopyridine is dissolved in diethyl ether, and triethylamine is added thereto; after several minutes, carbon disulfide is added; after reacting for a certain time, bromoethyl cyanide is added thereto; the resultant mixture is stirred for several hours at room temperature; then the reaction mixture is worked up according to the routine methods, such as purification by column chromatography, to obtain the desired target product.

When group A in the compounds of the present invention is 2-furyl, m=1, and the methyldithiocarbamate moiety is a cyclic group, the compounds may be synthesized by the following route: 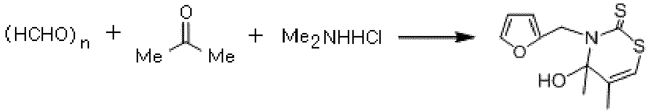 wherein, the furyl may have substituents as described in the definition of the general formula above.

Specifically, the preparation method is as follows: a mixed solution of paraformaldehyde, acetone, and dimethylamine hydrochloride in water and isopropanol is reacted for several hours under refluxing and stirring; then the reaction mixture is worked up with 50% NaOH; the concentrated organic phase is directly reacted, without further purification, with carbon disulfide and furfurylamine to obtain the above compounds in which the methyldithiocarbamate moiety is a cyclic group. According to the above process, other compounds of the general formula (I) in which the methyldithiocarbamate moiety is a cyclic group may be prepared.

The preparation of the pharmaceutically acceptable salts of the compounds of the general formula (I) of the present invention may be accomplished during the preparation of these compounds; and alternatively, after the compounds are prepared, the salts can be prepared by reacting the compounds with the corresponding acid according to routine methods.

Another objective of the present invention is to provide a pharmaceutical composition, the composition being a tyrosine kinase inhibitor which can used for treating diseases medicated by protein tyrosine kinase, such as for the treatment of tumor, especially breast cancer, liver cancer, non-small cell lung cancer, gastric cancer, colon cancer, leukaemia, nasal cancer or the like. The composition contains the compounds of the general formula (I) or the pharmaceutically acceptable salts thereof as an active ingredient and optionally contains pharmaceutical carrier(s).

Specifically, the composition contains a therapeutically effective amount of the compounds of general formula (I) of the present invention or their pharmaceutically acceptable salts, and one or more pharmaceutical carrier(s). Preferably, of the total weight of the composition, the content of the active ingredient in the composition of the present invention is 0.5%-99%, and the content of the pharmaceutical carrier(s) is 1%-99.5%.

The composition of the present invention can be formulated into various conventional pharmaceutical dosage forms, such as for oral administration or parenteral administration, said form for parenteral administration such as various forms for injection administration, topical administration, inhalation administration, rectal administration or implantable administration.

The form suitable for oral administration is such as tablets, capsules, granules or other pharmaceutically acceptable liquid preparations such as solutions, emulsions, suspensions or the like. The preferred oral preparation is tablets which can be formulated into film-coated, enterosoluble, sustained-release or quantitative-release form.

For preparing suitable dosage form, one or more pharmaceutical carrier(s) as required can be added into the active ingredient, said pharmaceutical carriers including various routine pharmaceutical a, such as excipients, fillers, diluents, disintegrants, surfactants, wetting agents, preservatives, sweetening agents, pigment and the like.

Depending on the type and the severity of diseases, as well as the conditions of patients such as sex, age, body weight and the like, appropriate dosage form and applied dosage are selected, where generally, the applied dosage for an adult is 1-200mg/kg body weight/day, preferably 1-50mg/kg body weight/day.

The pharmaceutical composition of the present invention and various dosage forms thereof can be prepared according to the routine methods known in the pharmaceutical field.

Another objective of the present is to provide a pharmaceutical use of the compounds of the general formula (I) or their pharmaceutically acceptable salts, and the present invention discloses the use of aforesaid compounds of general formula (I) or their pharmaceutically acceptable salts, and the pharmaceutical compositions containing the same in the manufacture of tyrosine kinase inhibitor, especially in manufacture of anti-tumor drugs. The anti-tumor drugs are especially suitable for treating tumors, such as breast cancer, liver cancer, non-small cell lung cancer, gastric cancer, colon cancer, leukaemia, nasal cancer or the like.

By screening the activity of the compounds of the present invention, including anti-tumor test in vitro and tyrosine kinase inhibition activity screening assays, the obtained results show that, the compounds of the general formula (I) of the present invention or their pharmaceutically acceptable salts have excellent protein tyrosine kinase inhibition activity and anti-cancer activity, and are expected to be developed as a kind of anti-cancer drug with novel structural types.

Another objective of the present invention is to provide a method for treating tumor, including administrating a therapeutically effective amount of the compounds of general formula (I) or their pharmaceutically acceptable salts, or the pharmaceutical composition described above to a patient in need thereof.

### DETAILED DESCRIPTION OF THE INVENTION

The following detailed embodiments are used for further illustration of the technical solutions of the present invention, wherein the examples listed are only intended to illustrate the present invention, without limiting the scope of the present invention in any way.

### Example 1: Preparation of 3-(furan-2-ylmethyl)-4-hydroxy-1,3-thiazinane-2-thione (compound 1)

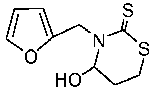

Furfurylamine (4 mmol) and carbon disulfide (5 mmol) were added into water (15 mL) in sequence, then triethylamine (8 mmol) was added. After the mixture was reacted with stirring for 10 min at room temperature, the solution turned light yellow, then acrolein (4 mmol) was slowly added dropwise, and white opacity was observed in a short time. After stirring for another 1 h, TLC monitored that the spot of the raw material disappeared, and the reaction was stopped. 20 mL of water was added, followed by extraction with EtOAc (15 mL × 3). The organic phase was combined, washed with water and saturated saline in sequence, dried over anhydrous MgSO₄. The organic phase was concentrated, and directly purified by pressurized column chromatography with EtOAc:petroleum ether as eluting agent. A light yellow solid was obtained and recrystallized, then a light yellow crystal was obtained. Yield: 34%, melting point: 42-43°C. The compound may turn dark slowly when exposed to air for a long time, so sealed storage is recommended.
¹HNMR (300MHz, CDCl₃): δ2.20 (m, 1 H), 2.45 (m, 1 H), 2.63 (m, 1 H), 3.39-3.54 (m, 2H), 5.02-5.07(d, 1 H), 5.38 (s, 1 H), 5.71-5,76 (m, 2H), 6.38-6.51 (bs, NH), 7.39 (m, 1 H). Anal Calcd. for C₉H₁₁NO₂S₂: C, 47.14; H, 4.83; N, 6.11. Found: C, 47.43; H, 5.14; N, 5.90.

### Example 2: Preparation of 2-(methoxycarbonyl)ethyl (furan-2-ylmethyl)dithiocarbamate (compound 2)

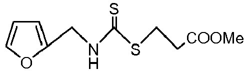

The preparation method of the compound was nearly identical to that described in Example 1, except for using methyl acrylate instead of acrolein. The target compound obtained was a white crystal. Yield: 81%, melting point: 50-52°C.
¹HNMR (300MHz, CDCl₃): δ 2.79 (t, 2H, J = 6.6 Hz); 3.53 (t, 2H, J = 6.6 Hz); 3.70 (s, 3H); 4.87 (s, 2H); 6.33-6.36 (m, 2H); 7.18 (bs, NH); 7.39 (s, 1 H). Anal Calcd. for C₁₀H₁₃NO₃S₂: C, 46.31; H, 5.05; N, 5.40. Found: C, 46.43; H, 5.24; N, 5.70.

### Example 3: Preparation of 2-cyanoethyl (furan-2-ylmethyl)dithiocarbamate (compound 3)

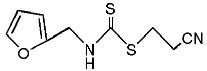

The preparation method of the compound was nearly identical to that described in Example 1, except for using 2-bromoethyl cyanide instead of acrolein. The target compound obtained was a white crystal. Yield: 61%, melting point: 53-54°C.
¹HNMR (300MHz, CDCl₃): δ 2.89 (t, 2H, J = 6.9Hz), 3.56 (t, 2H, J = 6.9 Hz), 4.88 (s, 2H), 7.12 (bs, NH), 7.33-7.41 (m, 3H). Anal Calcd. for C₈H₈N₂OS₂: C, 45.26; H, 3.80; N, 13.20. Found: C, 45.61; H, 3.97; N, 13.12.

### Example 4:

### 3-(furan-2-ylmethyl)-4-hydroxy-4,5-dimethyl-3,4-dihydro-2H-1,3-thiazine-2-thione (compound 4)

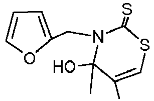

Firstly, acetone (1.2 mol), dimethylamine hydrochloride (0.6 mol), and water (150 mL) were added into 500 mL round-bottom flask. Paraformaldehyde (0.8 mol) and isopropanol (15 mL) were added under stirring at room temperature. Then the mixture was reacted for 6h under refluxing and stirring, before the reaction was stopped. After most of the solvent was evaporated under reduced pressure, an aqueous solution of 25g 50% NaOH was added slowly under ice-bath. The organic phase was separated, and the water phase was extracted with EtOAc (25 mL × 4). The organic phase was combined, dried over anhydrous Na₂SO₄, and used directly for the next step after being concentrated under reduced pressure.

Then, according to the same synthetic procedure as that in Example 1, the concentrated solution obtained from the above step was reacted with corresponding carbon disulfide and furfurylamine to provide the target compound as a white solid. Yield: 57%, melting point: 88-89°C.
¹HNMR (300MHz, CDCl₃): δ 2.37 (s, 3H), 3.10-3.15 (m, 3H), 3.59-3.62(m, 1H), 3.65-3.78 (m, 1 H), 5.12-5.17(d, 1 H), 5.47-5.52 (d, 1 H), 6.36-6.47 (m, 2H), 7.40 (m, 1 H). Anal Calcd. for C₁₁H₁₃NO₂S₂: C, 51.74; H, 5.13; N, 5.49. Found: C, 51.46; H, 5.67; N, 5.12.

### Example 5: Preparation of 2-sulfamoylethyl (furan-2-ylmethyl)dithiocarbamate (compound 5)

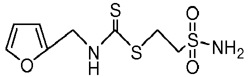

Under ice-bath, the catalyst aluminum oxide (4.0 mmol) was added into a solution of 2-chloroethanesulfonyl chloride (4.0 mmol) in dioxane (30 mL). The ammonia gas was conducted slowly under stirring. Formation of white mist was observed during the reaction. After the reaction was kept under stirring for 2h, the mixture was filtered under pump. After part of the solvent was evaporate under reduced pressure, 50 mL water was added, and extraction with EtOAc (25 mL × 3) was carried out. The organic phase was combined, dried over anhydrous Na₂SO₄, and concentrated under reduced pressure to afford ethenesulfonamide as a light yellow liquid. Without further purification, the light yellow liquid was reacted with furfurylamine and carbon disulfide to afford the corresponding product as an oil. Total yield: 35%.
¹HNMR (300MHz, CDCl₃): δ 3.51-3.55 (m, 2H), 4.08-4.13 (m, 2H), 4.74(s, 2H), 4.88-4.90(m, 2H), 6.35-6.38(m, 2H), 7.18(bs, NH), 6.36-6.47(m, 2H), 7.40-7.41 (m, 1 H). Anal Calcd. for C₈H₁₂N₂O₃S₃: C, 34.27; H, 4.31; N, 9.99. Found: C, 34.35; H, 4.56; N, 10.13.

### Example 6: Preparation of 2-boronoethyl (furan-2-ylmethyl)dithiocarbamate (compound 6)

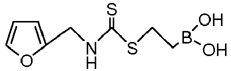

At -76°C, using anhydrous THF as solvent, 1,2-dibromoethane was reacted with n-BuLi to afford 2-bromoethyl lithium under the protection of N₂. Without purification, the mixture was directly reacted with trimethyl borate to afford corresponding dimethyl (2-bromoethyl)boronate. Then the resultant mixture was treated with a saturated solution of HCl in ether, to afford the key intermediate (2-bromoethyl)boric acid. Without further purification, according to the same procedure in Example 1, the intermediate was directly reacted with furfurylamine and carbon disulfide to afford the target compound as a light yellow solid. Total yield: 19%, melting point: 73-75°C.
¹HNMR (300MHz, CDCl₃): δ 3.51-3.55 (m, 2H), 4.08-4.13 (m, 2H), 4.74 (s, 2H), 4.88-4.90 (m, 2H), 6.35-6.38 (m, 2H), 7.18 (bs, NH), 6.36-6.47 (m, 2H), 7.40-7.41 (m, 1 H). Anal Calcd. for C₈H₁₂BN₂O₃S₃: C, 34.27; H, 4.31; N, 9.99. Found: C, 34.35; H, 4.56; N, 10.13.

### Example 7: Preparation of 2-(methylsulfinyl)ethyl (furan-2-ylmethyl)dithiocarbamate (compound 7)

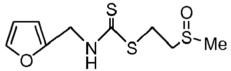

1,2-dibromoethane (10 mmol) and NaOH (30 mmol) were added into water (10 mL). TBAB (2 mmol), a phase transfer catalyst, was added under stirring. A solution of S-methyl-isothiourea sulfate (10 mmol) in water (10 mL) was added after stirring for another 10 min. The reaction is continued under stirring for another 1 h; then 15 mL CH₂Cl₂ was added, followed by overnight reaction. The dichloromethane layer was separated from the reaction system, and 80% formic acid solution (1.5 g) was added. 30% H₂O₂ (2.0 g) was added dropwise under stirring at room temperature, and then the solution was kept reacting under stirring for 4h. A color change of the solution from colorless to light yellow was observed. The reaction was stopped, allowed to stand to separate into layers. The organic phase was adjusted to around pH7 using an aqueous solution of 5% Na₂CO₃, followed by extraction with CH₂Cl₂. The organic phase was dried over anhydrous Na₂SO₄, and then was concentrated under reduced pressure to provide the crude product 2-bromoethyl methyl sulfoxide. Without further purification, the crude product was directly used in the next step. According to the same procedure as Example 1, it was reacted with furfurylamine and carbon disulfide to afford the target compound as a white solid Yield: 65%, melting point: 43-45°C.
¹HNMR (300MHz, CDCl₃): δ 1.55 (s, 3H), 3.25 (t, 2H), 4.05 (t, 2H), 4.66 (bs, NH), 4.96 (s, 2H), 6.29-6.40 (m, 2H), 6.36-6.47(m, 2H), 7.36-7.41 (m, 1 H). Anal Calcd. for C₉H₁₃NO₂S₃: C, 41.04; H, 4.97; N, 5.32. Found: C, 40.96; H, 4.85; N, 5.12.

### Example 8: Preparation of 2-(benzylsulfinyl)ethyl (furan-2-ylmethyl)dithiocarbamate (compound 8)

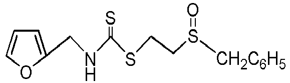

The preparation method of the compound was nearly identical to that described in Example 7, except for using S-benzyl-isothiourea sulfate instead of S-methyl-isothiourea sulfate. The compound was obtained as white solid. Yield: 77%, melting point: 51-52°C.
¹HNMR (300MHz, CDCl₃): δ 3.28 (m, 2H), 4.03 (m, 2H), 4.68 (bs, NH), 4.94 (s, 2H), 6.29-6.40 (m, 2H), 7.18(bs, NH), 6.36-6.47(m, 2H), 7.37-7.45(m, 6H). Anal Calcd. for C₁₅H₁₇NO₂S₃: C, 53.07; H,5.05; N, 4.13. Found: C, 53.26; H, 5.13; N, 4.02.

### Examples 9-29: Preparation of compounds 9-29

The preparation method of the compounds was nearly identical to that of furylalkylamine derivatives described in Examples 1-8, except for using the corresponding pyridylalkylamines instead of furylalkylamines. The compounds prepared and the data of structure characterization are as follows:

### 4-hydroxy-3-(pyridin-3-ylmethyl)-1,3-thiazinane-2-thione (compound 9)

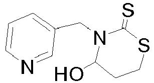

Light yellow solid, yield: 58%, melting point: 56-57°C.
¹HNMR (CDCl₃, 300 MHz): δ 2.22 (m, 1 H), 2.48 (m, 1 H), 2.66 (m, 1 H), 3.40-3.56 (m, 2H), 5.03-5.08 (d, 1 H), 5.38 (s, 1 H), 6.21-6.32(m, 1 H), 6.38-6.51 (bs, NH), 7.39 (m, 3H). Anal Calcd. for C₁₀H₁₂N₂OS₂: C, 49.97; H, 5.03; N, 11.66. Found: C, 49.42; H, 5.16; N, 11.23.

### 2-(methoxycarbonyl)ethyl (pyridin-3-ylmethyl)dithiocarbamate (compound 10)

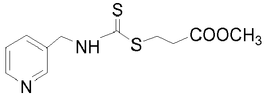

White crystal, yield: 72%, melting point: 50-52°C.
¹HNMR (CDCl₃, 300 MHz): δ 2.80 (t, 2H, J = 6.9 Hz), 3.53 (t, 2H, J = 6.9 Hz), 3.70 (s, 3H), 4.94 (s, 2H), 7.28-7.32 (m, 1 H), 7.70 (m, 1 H), 7.73 (bs, NH), 8.53-8.56 (m, 2H). Anal Calcd. for C₁₁H₁₄N₂O₂S₂: C, 48.87; H, 5.22; N,1 0.36. Found: C, 48.55; H, 5.24; N, 10.70.

### 2-cyanoethyl (pyridin-3-ylmethyl)dithiocarbamate (compound 11)

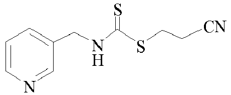

White solid, yield: 77%, melting point: 58-60°C.
¹HNMR (CDCl₃, 300 MHz): δ 2.87 (t, 2H, J = 6.6Hz), 3.54 (t, 2H, J = 6.6 Hz), 4.88 (s, 2H), 7.12 (bs, NH), 7.23 (m, 1 H), 7.53-7.64 (m, 3H). Anal Calcd. for C₁₀H₁₁N₃S₂: C, 50.60; H, 4.67; N, 17.70. Found: C, 50.72; H, 4.93; N, 17.62.

### 4-hydroxy-4,5-dimethyl-3-(pyridin-3-ylmethyl)-3,4-dihydro-2H-1,3-thiazine-2-thione (compound 12)

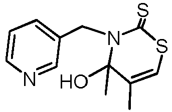

White solid, yield: 41%, melting point: 71-72°C.
¹HNMR (CDCl₃, 300 MHz): δ 2.38 (s, 3H), 3.12-3.16 (m, 3H), 3.59-3.64 (m, 1H), 3.65-3.78 (m, 1 H), 5.12-5.17 (d, 1 H), 5.47-5.52 (d, 1 H), 6.46-6.48 (m, 1 H), 7.40-7.42 (m, 3H). Anal Calcd. for C₁₂H₁₄N₂OS₂: C, 54.11; H, 5.30; N, 11.52 Found: C, 54.47; H, 5.66; N, 11.33.

### 2-sulfamoylethyl (pyridin-3-ylmethyl)dithiocarbamate (compound 13)

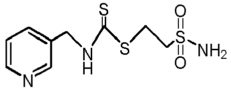

Oil, yield: 41%.
¹HNMR (CDCl₃, 300 MHz): δ 3.52-3.55 (m, 2H),4.10-4.15 (m, 2H), 4.76 (s, 2H), 4.84-4.91 (m, 2H), 6.25-6.35 (m, 2H), 7.18 (bs, NH), 6.36-6.47 (m, 1 H), 7.40-7.42 (m, 3H). Anal Calcd. for C₉H₁₃N₃O₃S₃: C, 37.09 H, 4.50; N, 14.42 Found: C, 37.35; H, 4.58; N, 14.16.

### 2-boronoethyl (pyridin-3-ylmethyl)dithiocarbamate (compound 14)

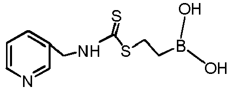

White solid, yield: 48%, melting point: 49-50°C.
¹HNMR (CDCl₃, 300 MHz): δ 3.49-3.51 (m, 2H),4.10-4.15 (m, 2H), 4.74 (s, 2H), 4.88-4.90 (m, 2H), 6.35-6.38 (m, 2H), 7.20 (bs, NH), 6.36-6.47 (m, 1 H), 7.40-7.41 (m, 3H). Anal Calcd. for C₉H₁₃BN₂O₂S₂: C, 42.20 H, 5.12; N, 10.94 Found: C, 42.45; H, 5.56; N, 10.13.

### 2-(methylsulfinyl)ethyl (pyridin-3-ylmethyl)dithiocarbamate (compound 15)

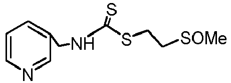

Oil, yield: 43%.
¹HNMR (CDCl₃, 300 MHz): δ 1.65 (s, 3H), 3.26 (t, 2H), 4.05 (t, 2H), 4.86 (bs, NH), 4.96 (s, 2H), 6.29-6.40 (m, 1 H), 7.16-7.31 (m, 3H). Anal Calcd. for C₁₀H₁₄N₂OS₃: C, 43.77; H, 5.14; N, 10.21. Found: C, 43.76; H, 5.35; N, 10.37.

### 2-(benzylsulfinyl)ethyl (pyridin-3-ylmethyl)dithiocarbamate (compound 16)

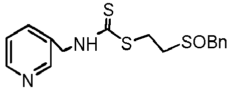

White solid, yield: 51 %, melting point: 64-66°C.
¹HNMR (CDCl₃, 300 MHz): δ 3.27 (m, 2H), 4.12 (m, 2H), 4.89 (bs, NH), 4.94 (s, 2H), 6.29-6.40 (m, 2H), 7.18(bs, NH), 6.36-6.47(m, 1 H), 7.37-7.45(m, 5H), 7.51-7.53(m, 3H). Anal Calcd. for C₁₇H₁₈N₂O₂S₂ : C, 58.93; H,5.24; N, 8.09. Found: C, 58.76; H, 5.16; N, 8.21.

### 2-cyanoethyl [(4-pivalamidopyridin-3-yl)methyl]dithiocarbamate (compound 17)

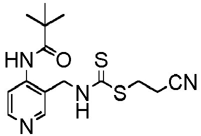

White solid, yield: 63%, melting point: 122-124°C.
¹HNMR (CDCl₃, 400 MHz): δ 8.47 (d, 1H, *J*= 5.6 Hz), 8.40 (m, 2H), 8.12 (d, 2H, *J* =5.6 Hz), 4.94 (d, 2H, *J*= 4.8 Hz), 3.54 (t, 2H, *J* =6.8 Hz), 2.86 (t, 2H, *J*= 6.8 Hz), 1.36 (s, 9H).

### 2-cyanoethyl (quinolin-3-ylmethyl)dithiocarbamate (compound 18)

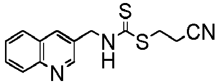

White solid, yield: 72%, melting point: 120-122°C.
¹HNMR (CDCl₃, 400 MHz): δ 9.14 (m, 1 H), 8.78 (s, 1 H), 8.05 (m, 2H), 7.70 (m, 2H), 7.52 (t, 1 H, J = 5.7 Hz), 5.07 (d, 2H, J =5.6 Hz), 3.53 (t, 2H, J= 6.8 Hz), 2.89 (t, 2H, J = 6.8 Hz). ¹³CNMR(CDCl₃, 100 MHz):δ196, 150, 147, 136, 130, 129, 128.7, 127.8, 127.6, 127, 118, 49, 30, 18.

### 2-cyanoethyl [2-(pyridin-3-yl)ethyl]dithiocarbamate (compound 19)

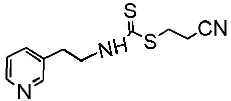

Light yellow solid, yield: 74%, melting point: 82-84°C.
¹HNMR (CDCl₃, 400 MHz): δ 8.50 (m, 1 H), 8.39 (m, 2H), 7.56 (d, 1 H, J = 7.6 Hz), 7.25 (dd, 1 H, J = 7.6, 5.2 Hz), 4.04 (dd, 2H, J = 12.8, 6.4 Hz), 3.53 (t, 2H, J = 6.8 Hz), 3.04 (t, 2H, J= 6.4 Hz), 2.87 (t, 2H, J= 6.8 Hz).
¹³CNMR (CDCl₃, 100 MHz): δ 196, 149, 147, 136, 134, 123, 118, 47, 31, 30, 18.

### 2-cyanoethyl [(2-methoxypyridin-3-yl)methyl]dithiocarbamate (compound 20)

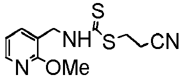

White solid, yield: 67%, melting point: 88-92°C.
¹HNMR (CDCl₃, 400 MHz): δ 8.10 (dd, 1 H, J= 1.6, 3.6 Hz), 7.71 (m, 1 H), 7.59 (dd, 1 H, J =1.6, 7.2 Hz), 6.83 (dd, 1 H, J= 3.6, 7.2 Hz), 4.86 (d, 2H, J= 5.2 Hz), 3.90 (s, 3H), 3.47 (t, 2H, J= 6.8 Hz), 2.82 (t, 2H, J= 6.8 Hz).
¹³CNMR(CDCl₃, 100 MHz):δ 195, 162, 146, 139, 118, 118, 116, 54, 46, 30, 18.

### 2-cyanoethyl [(6-methoxycarbonylpyridin-3-yl)methyl]dithiocarbamate (compound 21)

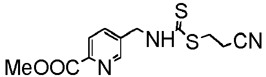

White solid, yield: 30%, melting point: 106-112°C.
¹HNMR (d⁶DMSO, 400 MHz): δ 10.72(m, 1 H), 8.63(d, 1 H, J=1.6 Hz), 8.03(d, 1 H, *J*= 8.0 Hz), 7.85(dd, 1 H, *J*= 1.6, 8.0 Hz), 4.93(d, 2H, *J*= 5.2 Hz), 3.85(s, 3H), 3.47(t, 2H, *J*= 6.8 Hz), 2.90(t, 2H, *J*= 6.8 Hz).
¹³CNMR (d⁶DMSO, 100 MHz): δ 196, 165, 150, 147, 137, 136, 125, 118, 53, 47, 30, 18.

### 2-cyanoethyl [(4,6-dimethylpyridin-3-yl)methyl]dithiocarbamate (compound 22)

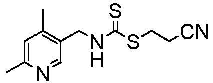

Light yellow solid, yield: 76%, melting point: 138-142°C.
¹HNMR (d⁶DMSO, 400 MHz): δ 8.22(s, 1 H), 7.04(s, 1 H), 4.75(s, 2H), 3.44(t, 2H, J = 6.8 Hz), 2.87(t, 2H, J = 6.8 Hz), 2.37(s, 3H), 2.22(s, 3H).
¹³CNMR (d⁶DMSO, 100 MHz): δ 195, 157, 149, 146, 128, 125, 119, 46, 30, 24, 19, 18.

### 2-cyanoethyl [(5-methoxypyridin-3-yl)methyl]dithiocarbamate (compound 23)

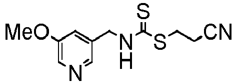

White solid, yield: 73%, melting point: 86-90°C.
¹HNMR (CDCl₃, 400 MHz): δ 8.22(s, 1 H), 8.14(s, 1 H), 8.02(m, 1 H), 7.21 (s, 1 H), 4.92(d, 2H, J = 5.6 Hz), 3.81 (s, 3H), 3.53(t, 2H, J= 6.8 Hz), 2.87(t, 2H, J = 6.8 Hz).
¹³CNMR (CDCl₃, 100 MHz): δ 196, 149, 149, 146, 122, 122, 118, 49, 30, 18, 17.

### 2-cyanoethyl [(5-bromopyridin-3-yl)methyl]dithiocarbamate (compound 24)

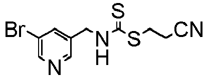

Light yellow solid, yield: 71 %, melting point: 102-108°C.
¹HNMR (CDCl₃, 400 MHz): δ 8.53(d, 1 H, J = 1.6 Hz), 8.49(d, 1 H, J = 1.6 Hz), 8.46(d, 1 H, J =1.6 Hz), 7.84(s, 1 H), 4.91 (d, 2H, J = 5.6 Hz), 3.51 (t, 2H, J = 6.8 Hz), 2.86(t, 2H, J = 6.8 Hz).
¹³CNMR (CDCl₃, 100 MHz): δ 196, 156, 150, 147, 138, 134, 120, 47, 31, 18.

### 2-cyanoethyl {[6-(furan-2-yl)pyridin-3-yl]methyl}dithiocarbamate (compound 25)

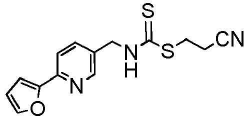

Light yellow solid, yield: 78%, melting point: 118-120°C.
¹HNMR (CDCl₃, 400 MHz):δ 8.43(m, 2H), 7.66(dd, 1 H, J= 1.6, 7.6 Hz), 7.58(d, 1 H, J = 7.6 Hz), 7.50(s, 1 H), 7.00(d, 1 H, J = 3.2 Hz), 6.51 (dd, 1 H, J = 1.6, 3.2 Hz), 4.89(d, 2H, J = 5.2 Hz), 3.51 (t, 2H, J = 6.8 Hz), 2.86(t, 2H, J = 6.8 Hz).
¹³CNMR (CDCl₃, 100 MHz): δ 196, 153, 149, 149, 143, 137, 130, 118, 118, 112, 109, 48, 31, 18.

### 2-cyanoethyl [(4-methyl-3,4,4a,8a-tetrahydro-2H-pyrido[3,2-b][1,4]oxazin-7-yl)-methyl]dithiocarbamate (compound 26)

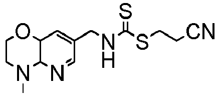

Light yellow solid, yield: 79%, melting point: 117-118°C.
¹HNMR (CDCl₃, 400 MHz): δ 8.63(s, 1H), 7.50(s, 1H), 6.73(s, 1H), 4.57(d, 2H, *J* = 4.0 Hz), 4.15(t, 2H, *J* = 3.6 Hz), 3.45(t, 2H, *J* = 6.8 Hz), 3.37(t, 2H, *J* = 3.6 Hz), 3.02(s, 3H), 2.81 (t, 2H, *J* = 6.8 Hz).
¹³CNMR (CDCl₃, 100 MHz): δ 194, 148, 139, 139, 121, 120, 118, 64, 49, 48, 36, 30, 19.

### 2-cyanoethyl [(6-phenoxypyridin-3-yl)methyl]dithiocarbamate (compound 27)

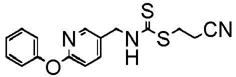

Oil, yield: 33%.
¹HNMR (CDCl₃, 400 MHz): δ 8.13(s, 0.5H), 8.05(s, 0.5H), 7.73(m, 1 H), 7.41 (t, 2H,J = 7.6 Hz)7.22(t,1H, *J* = 7.2 Hz), 7.11(d, 1 H, *J* = 7.6 Hz), 6.90(d, 1 H, *J* = 7.6 Hz), 5.28(m, 1 H), 4.83(m, 1 H), 3.50(t, 2H, *J* = 6.8 Hz), 2.83(t, 2H, *J* = 6.8 Hz).
¹³CNMR (CDCl₃, 100 MHz): δ 197, 154, 147, 140, 130, 125, 121, 121, 118, 111, 111, 56, 32,18.

### 2-cyanoethyl (pyridin-4-ylmethyl)dithiocarbamate (compound 28)

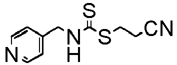

Oil, yield: 69%.
¹HNMR (CDCl₃, 400 MHz): δ 10.27(m, 1 H), 8.34(d, 2H, *J* = 5.6 Hz), 7.16(d, 2H, *J* = 5.6 Hz), 4.88(d, 2H, *J* = 5.2 Hz), 3.43(t, 2H, *J* = 6.8 Hz), 2.78(t, 2H, *J* = 6.8 Hz)

### 2-cyanoethyl N-methyl-(pyridin-3-ylmethyl)dithiocarbamate (compound 29)

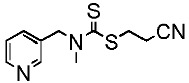

Oil, yield: 72%.
¹HNMR (CDCl₃, 400 MHz): δ 8.49(s, 2H), 7.57(m, 1H), 7.23(m, 1H), 5.23(s, 1.45H), 4.90(s, 0.45H), 3.50(t, 2H, *J* = 6.8 Hz), 3.36(s, 0.85H), 3.20(s, 2.02H), 2.88(t, 2H, *J* = 6.8 Hz).
¹³CNMR (CDCl₃, 100 MHz): δ 197, 149, 149, 135, 131, 124, 118, 57, 39, 32, 18.

### Example 30: Preparation of 2-cyanoethyl pyridin-3-yldithiocarbamate (compound 30)

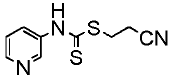

3-aminopyridine (1 mmol) was dissolved in diethyl ether (25 mL), and triethylamine (1 mmol) was added thereto. Several minutes later, carbon disulfide (4 mmol) was added. After a 20 minute reaction, 2-bromoethyl cyanide (1 mmol) was added. The mixture was stirred for 24h at room temperature; then, the mixture was worked up according to the routine method to obtain the desired target compound.

White solid, yield: 20%, melting point: 80-82°C.
¹HNMR (CDCl₃, 400 MHz): δ 10.81 (s, 1 H), 8.68(d, 1 H, J = 1.6 Hz), 8.51 (d, 1 H, J = 4.4 Hz), 8.27(s, 1 H), 7.38(dd, 1 H, J = 1.6, 4.4 Hz), 3.54(t, 2H, J= 6.8 Hz), 2.88(t, 2H, J = 6.8 Hz).

### Examples 31-33: Preparation of compounds 31-33

The preparation method of the compounds was nearly identical to that of furylalkylamine derivatives described in Example 3, except for using the corresponding pyridylalkylamines instead of furylalkylamines. The compounds prepared and the data of structure characterization are as follows:

### 2-cyanoethyl [phenyl(pyridin-3-yl)methyl]dithiocarbamate (compound 31)

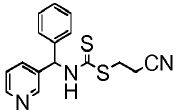

White solid, yield: 65%, melting point: 118-120°C.
¹HNMR (CDCl₃, 400 MHz): δ 9.46(d, 1 H, J = 6.8 Hz), 8.36(d, 2H, J = 6.8 Hz), 7.57(d, 1 H, J = 6.8 Hz), 7.36(m, 3H), 7.23(m, 3H), 6.94(d, 1 H, J = 6.8 Hz), 3.51 (t, 2H, J = 6.8 Hz), 2.85(t, 2H, J = 6.8 Hz).
¹³CNMR (CDCl₃, 100 MHz): δ 195, 149, 148, 139, 136, 129, 128, 128, 124, 118, 62, 31, 18.

### 2-cyanoethyl [1-(pyridin-3-yl)ethyl]dithiocarbamate (compound 32)

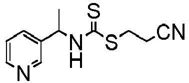

Oil, yield: 63%.
¹HNMR (CDCl₃, 400 MHz): δ 8.79(d, 1 H, *J* = 5.2 Hz), 8.60(m, 2H), 7.69(d, 1 H, *J* = 5.2 Hz), 7.36(s, 1 H), 5.80(s, 1 H), 3.50(t, 2H, *J* = 6.8 Hz), 2.85(t, 2H, *J* = 6.8 Hz).

### 2-cyanoethyl [(6-methoxypyridin-3-yl)methyl]dithiocarbamate (compound 33)

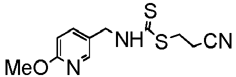

White solid, yield: 68%, melting point: 90-92°C.
¹HNMR (CDCl₃, 400 MHz): δ 8.10(d, 1 H, *J* = 2.4 Hz), 7.75(s, 1 H), 7.58(dd, 1 H, *J*= 2.4, 8.4 Hz), 6.72(d, 1 H, *J* = 8.4 Hz), 4.82(d, 2H, *J* = 4.8 Hz), 3.90(s, 3H), 3.50(t, 2H, *J* = 6.8 Hz), 2.85(t, 2H, *J* = 6.8Hz).
¹³CNMR (CDCl₃, 100 MHz): δ 197, 149, 149, 147, 123, 122, 118, 49, 30, 18, 17.

### Examples 34-42: Preparation of compounds 34-42

The preparation method of the compounds was nearly identical to that of furylalkylamine derivatives described in Example 3, except for using the corresponding heteroarylalkylamines instead of furylalkylamines as a raw material. The compounds prepared and the data of structure characterization are as follows:

### 2-cyanoethyl [(4-methyl-2-phenyloxazol-5-yl)methyl]dithiocarbamate (compound 34)

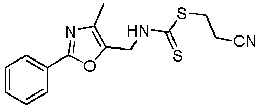

Yellow solid, yield: 85%, melting point: 221-224°C.
¹HNMR (CDCl₃, 400 MHz): δ 2.29 (s, 3H), 2.80-2.89 (t, J = 6.8 Hz, 2H), 3.53-3.59 (t, J = 6.8 Hz, 2H), 4.97 (s, 2H), 8.31 (bs, 1 H), 7.46-8.01 (m, 5H).

### 2-cyanoethyl [(5-methyl-1-phenyl-1H-pyrazol-4-yl)methyl]dithiocarbamate (compound 35)

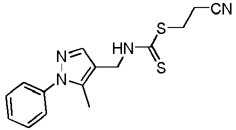

White crystal, yield: 87%, melting point: 135-138°C.
¹HNMR (CDCl₃, 400 MHz): δ 2.33 (s, 3H), 2.86-2.89 (t, *J* = 6.8 Hz, 2H), 3.52-3.56 (t, *J* = 6.8 Hz, 2H), 4.77 (s, 2H), 8.31 (bs, NH), 7.10 (bs, 1 H), 7.48-7.64 (m, 6H).

### 2-cyanoethyl (pyrimidin-5-ylmethyl)dithiocarbamate (compound 36)

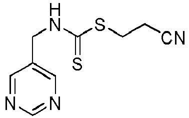

White crystal, yield: 81 %, melting point: 168-170°C.
¹HNMR (CDCl₃, 400 MHz): δ 2.88-2. 91 (t, *J* = 6.8 Hz, 2H), 3.57-3.61 (t, *J* = 6.8 Hz, 2H), 4.65 (s, 2H), 5.04 (m, 1 H), 5.35 (m, 1 H), 7.12-7.14 (m, 1 H).

### 2-cyanoethyl [(1,3-dimethyl-1H-pyrazol-5-yl)methyl]dithiocarbamate (compound 37)

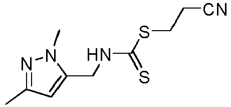

White solid, yield: 82%, melting point: 112-116°C.
¹HNMR (CDCl₃, 400 MHz): δ 2.23 (s, 3H), 2.85-2.88 (t, *J* = 6.8 Hz, 2H), 3.52-3.55 (t, *J* = 6.8 Hz, 2H), 3.76 (s, 3H); 4.90 (s, 2H), 6.04 (s, 1 H), 8.31 (bs, 1 H).

### 2-cyanoethyl (thiazol-2-ylmethyl)dithiocarbamate (compound 38)

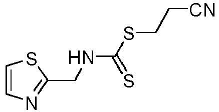

Yellow solid, yield: 78%, melting point: 76-78°C.
¹HNMR (CDCl₃, 400 MHz): δ 2.85-2.88 (t, J = 6.8 Hz, 2H), 3.52-3.56 (t, J = 6.8 Hz, 2H), 5.23 (s, 2H), 7.36-7.37 (s, 1 H), 7.77-7.78 (s, 1 H), 8.47 (bs, 1 H).

### 2-cyanoethyl (pyrazin-2-ylmethyl)dithiocarbamate (compound 39)

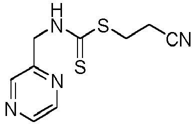

White crystal, yield: 69%, melting point: 96-99°C.
¹HNMR (CDCl₃, 400 MHz): δ 2.85-2.93 (t, J = 6.8 Hz, 2H), 3.55-3.63 (t, J = 6.8 Hz, 2H), 5.05 (s, 2H), 8.31 (bs, 1 H), 8.56-8.78 (m, 3H).

### 2-cyanoethyl [(5-phenyl-1 ,3,4-oxadiazol-2-yl)methyl]dithiocarbamate (compound 40)

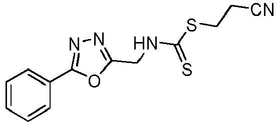

White solid, yield: 54%, melting point: 148-152°C.
¹HNMR (CDCl₃, 400 MHz): δ 2.85-2.89 (t, J = 6.8 Hz, 2H), 3.53-3.58 (t, J = 6.8 Hz, 2H), 5.24 (s, 2H), 7.50-7.55 (m, 3H), 8.02-8.04 (m, 2H), 8.23 (bs, 1 H).

### 2-cyanoethyl [(2-morpholinopyrimidin-5-yl)methyl]dithiocarbamate (compound 41)

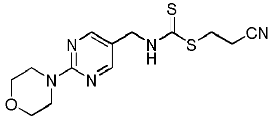

White crystal, yield: 90%, melting point: 151-154°C.
¹HNMR (CDCl₃, 400 MHz): δ 2.84-2.87 (t, J = 6.8 Hz, 2H), 3.50-3.53 (t, J = 6.8 Hz, 2H), 3.76-3.81 (m, 4H), 4.73 (s, 2H), 8.31-8.34 (s, 2H).

### 2-cyanoethyl [3-(pyridin-3-yl)propyl]dithiocarbamate (compound 42)

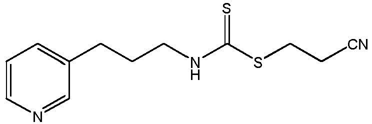

White crystal, yield: 77%, melting point: 81-83°C.
¹H NMR (CDCl₃, 400 MHz) : δ 2.01-2.04 (m, 2H), 2.70 (t, J = 6.8 Hz, 2H), 2.85-2.87 (m, 2H), 3.47-3.51 (t, J = 6.8 Hz, 2H), 3.76-3.80 (m, 2H), 7.24-7.26 (m, 1 H), 7.52-7.54 (m, 1 H), 8.43-8.46 (m, 2H), 8.84 (bs, 1 H).

### Example 43: Preparation of 2-cyanoethyl (pyridin-3-ylmethyl)dithiocarbamate hydrochloride (compound 43)

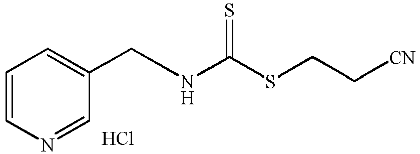

2-cyanoethyl (pyridin-3-ylmethyl)dithiocarbamate was dissolved in EtOAc, and a solution of 1.38 M hydrochloride in diethyl ether was added there to. After standing overnight, a large amount of precipitates appeared and was filtered, to afford a white solid. Melting point: 118-120°C.
¹HNMR (D₂O, 400 MHz): δ 2.84(s, 2H), 3.44(s, 2H), 5.00(s, 2H), 7.95(s, 1 H), 8.45(s, 1 H), 8.61 (s, 1 H), 8.67(s, 1 H).

### Example 44: Preparation of 2-cyanoethyl [2-(pyridin-3-yl)ethyl]dithiocarbamate hydrochloride (compound 44)

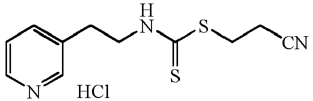

Usi n g 2-cyanoethyl [2-(pyridin-3-yl)ethyl]dithiocarbamate instead of 2-cyanoethyl (pyridin-3-ylmethyl)dithiocarbamate as a raw material, the target compound was prepared according to the same procedure as that in Example 43.
Yellow solid, melting point: 120-122°C.
¹HNMR (D₂O, 400 MHz): δ 8.57(m, 2H), 8.40(m, 1 H), 7.89(m, 1 H), 3.96(m, 2H), 3.36(m, 2H), 3.13(m, 2H), 2.77(m, 2H).

### Examples 45-46: Preparation of compounds 45-46

The preparation method of the compounds was nearly identical to that of furylalkylamine derivatives described in Examples 3, except for using the corresponding heteroarylalkylamines instead of furylalkylamines as a raw compound.

### 2-cyanoethyl [(5-phenyl-1 ,3,4-thiadiazol-2-yl)methyl]dithiocarbamate (compound 45)

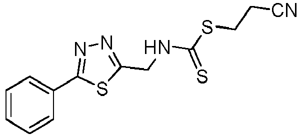

White solid, yield: 71 %, melting point: 156-158°C.
¹HNMR (CDCl₃, 400 MHz): δ 2.79-3.01 (t, *J* = 6.8 Hz, 2H), 3.43-3.67 (t, *J* = 6.8 Hz, 2H), 5.24 (s, 2H), 7.72-7.95 (m, 3H), 7.99-8.23 (m, 2H), 8.70 (bs, 1 H).

### 2-cyanoethyl [(1H-pyrrol-2-yl)methyl]dithiocarbamate (compound 46)

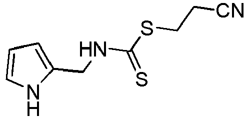

White solid, yield: 36%, melting point: 87-89°C.
¹HNMR (CDCl₃, 400 MHz): δ 2.81-2.97 (t, J = 6.8 Hz, 2H), 3.48-3.55 (t, J = 6.8 Hz, 2H),
5.24 (s, 2H), 5.83-5.91 (m, 1 H), 6.04-6.13 (m, 1 H), 6.59-6.66 (m, 1 H), 7.76 (bs, 1 H).

### Example 47: Preparation of 2-(butoxycarbonyl)ethyl (pyridin-3-ylmethyl)dithiocarbamate (compound 47)

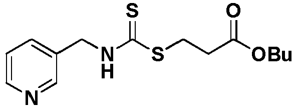

3-aminopyridine (1 mmol) was dissolved in acetone (25 mL), and anhydrous potassium phosphate (1 mmol) was added thereto. Several minutes later, carbon disulfide (1 mmol) was added. After a 20-minute reaction, n-butyl acrylate (1 mmol) was added. The mixture was stirred for 24 h at room temperature; then, the mixture was worked up according to the routine process to obtain the desired target compound.

White solid, yield: 88%, melting point: 54-56°C.
¹HNMR (CDCl₃,400 MHz): δ 0.88 (t, *J* = 7.4 Hz, 3H), 1.32 (dq, *J* = 14.6, 7.3 Hz, 2H), 1.46-1.63 (t, *J* = 6.8 Hz, 2H), 2.74 (t, *J* = 6.8 Hz, 2H), 3.48 (t, *J* = 6.8 Hz, 2H), 4.03 (t, *J* = 6.6 Hz, 2H), 4.90 (d, *J =* 5.3 Hz, 2H), 7.16-7.30 (m, 1 H), 7.67 (m, 1 H), 8.40 (m, 2H), 9.10 (bs, 1 H).

### Test example 1: Evaluation for the inhibitory effects of the compounds according to the present invention on the proliferation of different tumor cells (1)

The evaluation of the compounds according to the present invention on the inhibition rate of the proliferation of four different tumor cells, using the routine method for anti-tumor activity tests in the pharmaceutical filed, for example the method described in the following literature: *(*J. Immunol Method, 1983, 65, 55). The results are shown in Table 1.

**Table 1: The inhibitory effects of some compounds on the proliferation of four tumor cells at 10 µM (%)**

| Compounds NO. | The inhibitory effects on tumor cell proliferation | | | | | | | |
|---|---|---|---|---|---|---|---|---|
| | Model A | | Model B | | Model C | | Model D | |
| | Inhibitory rate(%) | Eval. | Inhibitory rate(%) | Eval. | Inhibitory rate(%) | Eval. | Inhibitory rate(%) | Eval. |
| 1 | 28.43 | - | 20.01 | - | 43.10 | - | 20.11 | - |
| 2 | 19.29 | - | 16.37 | - | -1.69 | - | 3.80 | - |
| 3 | 24.58 | - | 29.53 | - | 68.19 | + | 55.12 | + |
| 4 | 23.71 | - | 15.15 | - | 2.91 | - | 5.76 | - |
| 5 | 17.30 | - | 17.17 | - | 17.48 | - | 31.65 | - |
| 6 | 0.06 | - | 14.47 | - | 2.90 | - | 1.68 | - |
| 7 | 11.23 | - | 16.03 | - | -8.68 | - | 4.07 | - |
| 8 | 20.91 | - | 12.31 | - | 56.90 | + | 10.50 | - |
| 9 | 38.94 | - | 20.29 | - | 79.45 | + | 61.34 | + |
| 10 | 61.90 | - | 71.95 | + | 87.52 | ++ | 91.45 | ++ |
| 11 | 72.31 | + | 60.44 | + | 86.09 | ++ | 93.66 | ++ |
| 12 | 79.88 | + | 43.89 | - | 8.66 | - | 15.25 | - |
| 13 | -4.25 | - | 18.39 | - | 5.87 | - | 17.59 | - |
| 14 | 41.44 | - | 68.58 | + | 91.05 | ++ | 94.49 | ++ |
| 15 | 26.11 | - | 9.95 | - | -2.37 | - | 3.58 | - |
| 16 | 26.11 | - | 72.30 | + | 96.14 | ++ | 93.06 | ++ |

| | | | | | | | | |
|---|---|---|---|---|---|---|---|---|
| Eval.=Evaluation Note: 1. Test models: A: MTT method (HL-60 human leukaemia); B: SRB method (BGC-823 human gastric cancer); C: SRB method (Bel-7402 human liver cancer); D: SRB method (KB human nasopharyngeal cancer) 2. Test concentration: 10µM. | | | | | | | | |

### Test example 2: Evaluation of the inhibitory effects of the compounds according to the present invention on the proliferation of different tumor cells (2)

EC₅₀ values of the compounds according to the present invention on the proliferation of human leukaemia HL-60 cells and Bel-7402 cells were determined by using the following method, and the results are shown in Table 2.

Testing method for EC₅₀ values on the human leukaemia HL-60 cells: The human leukaemia HL-60 cells were cultured *in vitro.* The cells were collected after growth to logarithmic phase, and centrifugated at 1000 rpm for 5 min. The supernatant was discarded and the cells were suspended in an appropriate amount of medium. The cell concentration was adjusted to 1.2×10⁴/mL. The cells were seeded into 96-well culture plates in 90 µL per well. For each well of the drug treatment groups, 10 µL of medium-diluted drug was added, whereas for the blank control groups, the same amount of medium was added. Every testing drug was set up for three parallel wells, and also three control wells with only testing drugs (without cell) were set up. Negative control groups were medium containing 0.5% DMSO. After the plate was incubated in an incubator for 48 h, 10 µL of 5mg/mL MTT was added into each well, and then the plate was placed for 3 h at 37°C. 100 µL of ternary solution (5% SDS, 10mM HCl, 5% isopropanol) was added into each well, and the plate was kept at 37°C overnight. The absorbance (OD) was measured at 595 nm/620 nm. The EC₅₀ values were calculated by using Prism Graphpad statistical software.

Testing method for ED₅₀ values on the human Bel7402cell: After the cells were incubated with the addition of the drugs for 48 h, the plates were taken out. 50 µL of pre-cooled 500g/L trichloroacetic acid was added into each well. The plates were kept in 4°C refrigerator for 1 h after standing for 5 min, taken out, washed with deionized water for 5 times, and dried in the air. After the plates were completely dried, 100 µL of 0.4% SRB (formulated in 1% acetic acid) was added into each well. Following staining for 20 min, the staining solution was discarded, and the residue was washed with 1 % acetic acid for 5 times to remove the uncombined dye. After being dried in the air, the residue was dissolved in 150 µL of 10 mmol/L unbuffered Tris basic solution (pH=10.5), and oscillated in an oscillator for 5 min. The absorbance of each well at 490 nm was measured with TECAN enzyme mark instrument. The cells in background control plates were treated in the same way for measuring OD490. The EC₅₀ values were calculated by using Prism Graphpad statistical software.

**Table 2: ED₅₀ values of the compounds according to the present invention on the inhibition of the growth of HL-60 and Bel7402 cells (nmol)**

| Compounds NO. | HL-60 EC₅₀ (nmol) | Bel7402 EC₅₀ (nmol) |
|---|---|---|
| 11 | 1.462 | 27.23 |
| 17 | 0.4731 | 32.31 |
| 18 | 5.184 | 327.3 |
| 19 | 0.7962 | 18.75 |
| 20 | 0.8002 | 7.673 |
| 21 | 6.935 | 116.3 |
| 22 | 1.716 | 23.41 |
| 23 | 0.5572 | 10.27 |
| 24 | 4.909 | 20.54 |
| 25 | 4.778 | 21.15 |
| 26 | 82.20 | 96.39 |
| 27 | 0.5482 | 48.93 |
| 28 | 56.45 | 10.51 |
| 29 | 133.8 | 2.198 |
| 30 | 80.66 | 286.0 |
| 31 | 4.695 | 234.5 |
| 32 | 3.283 | 64.81 |
| 33 | 0.8126 | 108.9 |
| 34 | 7.88 | 17.95 |
| 35 | 6.05 | 24.94 |
| 37 | 4.17 | 4.263 |
| 38 | 108.60 | 295.6 |
| 39 | >1000 | >1000 |
| 40 | 14.20 | 25.66 |
| 41 | 5.03 | 22.39 |
| 42 | 4.82 | 3.481 |
| 45 | 11.77 | 18.65 |
| 46 | 282.50 | 226.7 |
| 47 | 25.42 | 6.355 |

### Test example 3: Evaluation of the inhibitory activities of the compounds according to the present invention on protein tyrosine kinase EGFR

The inhibitory activities of the compounds in examples 11, 16 and 47 of the present invention on protein tyrosine kinase EGFR were evaluated by the following method. The dual receptor tyrosine kinase inhibitor of EGFR and erbB2, Lapatinib, which is a small molecule and has been marketed, was selected as positive control. Clinical assays have proved that Lapatinib has a significant effect in the treatment of invasive, relapsed, inflammatory and brain metastatic breast cancer.

The experimental method was as follows:
Cell lines: MDA-MB-468 (cell line of EGFR over-expression), SK-BR-3 (cell line of erbB2 over-expression), and HCT 116 (as a control, cell line of low-expression of both EGFR and erbB2) were selected;
Positive control: Lapatinib;
Experimental operations: Human breast cancer SK-BR-3, MDA-MB-468 cells and human colon cancer HCT 116 cells were cultured *in vitro.* The cells were collected after growth to logarithmic phase, and centrifugated at 1000 rpm for 5 min. The supernatant was discarded, and the cells were suspended with an appropriate amount of medium. The cell concentration was adjusted to 3.5×10³/mL. Then the cells were seeded into 96-well culture plates in 100 µL per well, and placed in an incubator (37°C, 5% CO₂) to be incubated for 24h. The testing drugs were added, and the negative control group was added DMSO with the final concentration of 5%. Each group has three parallel wells. After being incubated in the incubator for 72h, 20 µL of 5mg/mL MTT was added into each well, and the plate was placed at 37°C for 4 h. 200 µL of DMSO was added into each well, and then the plate was oscillated in an oscillator for 30 min. The absorbance (OD) was measured at 492 nm/620 nm. The EC₅₀ values were calculated by using Prism Graphpad statistical software.

The experiment results are shown in Table 3.

**Table 3: The inhibitory activities of compounds 11, 16 and 47 on protein tyrosine kinase EGFR**

| Samples | SK-BR-3 (µM) | MDA-MB-468 (µM) | HCT 116 (µM) |
|---|---|---|---|
| Compound 11 | 2.840 | 9.537 | 10.41 |
| Compound 16 | 0.6549 | 0.9532 | 16.18 |
| Compound 47 | 4.461 | 12.79 | 7.835 |
| Lapatinib | 4.348 | 19.60 | 42.36 |

From the experiment results as above, it can be clearly seen that the compounds of the general formula (I) claimed by the present invention have better inhibitory activities on protein tyrosine kinase and anti-cancer activities than those of Lapatinib, and thus have a potential to be developed as a novel anti-tumor drugs with a novel structural type. Especially, when the group A is pyridyl and R³ is cyano, alkoxycarbonyl, borono or benzylsulfinyl, the compounds show better anti-tumor activities.

The embodiments of the present invention have been described in detail. It is obvious to the skilled in the art that various modifications and alternations can be made without departing from the fundamental spirit of the present invention. All these modifications and alternations are within the scope of the present invention, and their features are determined by the description as above.

## Claims

1. A dithiocarbamate compound represented by the general formula (I) or pharmaceutically acceptable salts thereof: 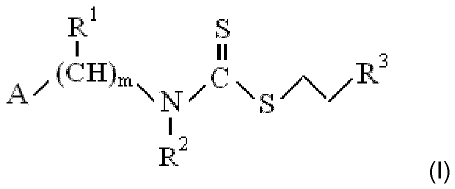 wherein:
A is substituted or unsubstituted, five- or six-membered heterocyclic group having one or more heteroatoms selected from nitrogen, oxygen and sulfur, wherein the heterocyclic group can be substituted with one or more substituents selected from halogen, C₁₋₄alkyl, C₁₋₄alkoxy, phenyl, phenoxy, furyl, morpholinyl/morpholino, C₁₋₆alkylamido and C₁₋₄alkoxycarbonyl; when the heterocyclic group is pyridyl, such pyridyl can be fused with benzene or heterocyclic group, such fused benzene or heterocyclic group is unsubstituted or substituted with C₁₋₄alkyl;
R¹ is H, phenyl, or C₁₋₄alkyl;
R² is H or C₁₋₄alkyl;
R³ is cyano, borono, C₁₋₄alkoxycarbonyl, aminosulfonyl, benzylsulfinyl, or C₁₋₄alkylsulfinyl;
or R² and R³ link together to form a saturated or unsaturated, five- or six-membered heterocyclic group containing nitrogen and sulfur atoms, and the heterocyclic group is unsubstituted or substituted with one or more hydroxy or C₁₋₄alkyl; and
m is an integer between 0 and 3.

2. The compound according to Claim 1, wherein the group A is substituted or unsubstituted heterocyclic group, is the heterocyclic group being selected from pyridyl, pyrimidinyl, pyrazinyl, furyl, oxazolyl, pyrazolyl, thiazolyl or oxadiazolyl; preferably the group A is substituted or unsubstituted pyridyl, or pyridyl fused with benzene or morpholine ring, the fused benzene or morpholine ring being unsubstituted or substituted with methyl.

3. The compounds according to Claim 2, wherein the C₁₋₄alkyl is methyl, the C₁₋₄alkoxy is methoxy, the C₁₋₄alkoxycarbonyl is methoxycarbonyl, and/or the C₁₋₆alkylamido is pentylamido.

4. The compound according to Claim 3, wherein the R³ group is cyano.

5. The compound according to any one of Claims 1-4, wherein the compound is:
3-(furan-2-ylmethyl)-4-hydroxy-1,3-thiazinane-2-thione (compound 1);
2-(methoxycarbonyl)ethyl (furan-2-ylmethyl)dithiocarbamate (compound 2);
2-cyanoethyl (furan-2-ylmethyl)dithiocarbamate (compound 3);
3-(furan-2-ylmethyl)-4-hydroxy-4,5-dimethyl-3,4-dihydro-2H-1,3-thiazine-2-thione (compound 4);
2-sulfamoylethyl (furan-2-ylmethyl)dithiocarbamate (compound 5);
2-boronoethyl (furan-2-ylmethyl)dithiocarbamate (compound 6);
2-(methylsulfinyl)ethyl (furan-2-ylmethyl)dithiocarbamate (compound 7);
2-(benzylsulfinyl)ethyl (furan-2-ylmethyl)dithiocarbamate (compound 8);
4-hydroxy-3-(pyridin-3-ylmethyl)-1,3-thiazinane-2-thione (compound 9);
2-(methoxycarbonyl)ethyl (pyridin-3-ylmethyl)dithiocarbamate (compound 10);
2-cyanoethyl (pyridin-3-ylmethyl)dithiocarbamate (compound 11);
4-hydroxy-4,5-dimethyl-3-(pyridin-3-ylmethyl)-3,4-dihydro-2H-1,3-thiazine-2-thione (compound 12);
2-sulfamoylethyl (pyridin-3-ylmethyl)dithiocarbamate (compound 13);
2-boronoethyl (pyridin-3-ylmethyl)dithiocarbamate (compound 14);
2-(methylsulfinyl)ethyl (pyridin-3-ylmethyl)dithiocarbamate (compound 15);
2-(benzylsulfinyl)ethyl (pyridin-3-ylmethyl)dithiocarbamate (compound 16);
2-cyanoethyl [(4-pivalamidopyridin-3-yl)methyl]dithiocarbamate (compound 17);
2-cyanoethyl (quinolin-3-ylmethyl)dithiocarbamate (compound 18);
2-cyanoethyl [2-(pyridin-3-yl)ethyl]dithiocarbamate (compound 19);
2-cyanoethyl [(2-methoxypyridin-3-yl)methyl]dithiocarbamate (compound 20);
2-cyanoethyl [(6-methoxycarbonylpyridin-3-yl)methyl]dithiocarbamate (compound 21);
2-cyanoethyl [(4,6-dimethylpyridin-3-yl)methyl]dithiocarbamate (compound 22);
2-cyanoethyl [(5-methoxypyridin-3-yl)methyl]dithiocarbamate (compound 23);
2-cyanoethyl [(5-bromopyridin-3-yl)methyl]dithiocarbamate (compound 24);
2-cyanoethyl {[6-(furan-2-yl)pyridin-3-yl]methyl}dithiocarbamate (compound 25);
2-cyanoethyl
[(4-methyl-3,4,4a,8a-tetrahydro-2*H*-pyrido[3,2-b][1,4]oxazin-7-yl)methyl]dithiocarbamate (compound 26);
2-cyanoethyl [(6-phenoxypyridin-3-yl)methyl]dithiocarbamate (compound 27);
2-cyanoethyl (pyridin-4-ylmethyl)dithiocarbamate (compound 28);
2-cyanoethyl N-methyl-(pyridin-3-ylmethyl)dithiocarbamate (compound 29);
2-cyanoethyl pyridin-3-yldithiocarbamate (compound 30);
2-cyanoethyl [phenyl(pyridin-3-yl)methyl]dithiocarbamate (compound 31);
2-cyanoethyl [1-(pyridin-3-yl)ethyl]dithiocarbamate (compound 32);
2-cyanoethyl [(6-methoxypyridin-3-yl)methyl]dithiocarbamate (compound 33);
2-cyanoethyl [(4-methyl-2-phenyloxazol-5-yl)methyl]dithiocarbamate (compound 34);
2-cyanoethyl [(5-methyl-1-phenyl-1*H*-pyrazol-4-yl)methyl]dithiocarbamate (compound 35);
2-cyanoethyl (pyrimidin-5-ylmethyl)dithiocarbamate (compound 36);
2-cyanoethyl [(1,3-dimethyl-1*H*-pyrazol-5-yl)methyl]dithiocarbamate (compound 37);
2-cyanoethyl (thiazol-2-ylmethyl)dithiocarbamate (compound 38);
2-cyanoethyl (pyrazin-2-ylmethyl)dithiocarbamate (compound 39);
2-cyanoethyl [(5-phenyl-1 ,3,4-oxadiazol-2-yl)methyl]dithiocarbamate (compound 40);
2-cyanoethyl [(2-morpholinopyrimidin-5-yl)methyl]dithiocarbamate (compound 41);
2-cyanoethyl [3-(pyridin-3-yl)propyl]dithiocarbamate (compound 42);
2-cyanoethyl (pyridin-3-ylmethyl)dithiocarbamate hydrochloride (compound 43);
2-cyanoethyl [2-(pyridin-3-yl)ethyl]dithiocarbamate hydrochloride (compound 44);
2-cyanoethyl [(5-phenyl-1,3,4-thiadiazol-2-yl)methyl]dithiocarbamate (compound 45);
2-cyanoethyl [(1H-pyrrol-2-yl)methyl]dithiocarbamate (compound 46); and
2-(butoxycarbonyl)ethyl (pyridin-3-ylmethyl)dithiocarbamate (compound 47).

6. A method for preparing the compounds according to any one of Claims 1-5 or the pharmaceutically acceptable salts thereof, the methods including: firstly, preparing the compounds of the general formula (I) according to the following route; 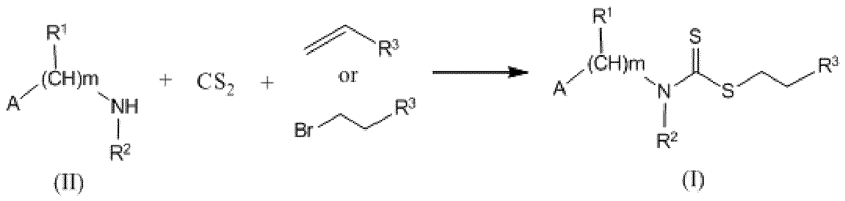 wherein each group in general formulae (I) and (II) are defined as in Claims 1-5;
if necessary, the pharmaceutically acceptable salts of the compounds can be prepared according to the routine salt-forming process in the chemical field.

7. A method for preparing the compounds according to any one of Claims 1-5 or the pharmaceutically acceptable salts thereof, wherein, when the group A of the compounds of the general formula (I) is substituted or unsubstituted pyridyl, and R³ is cyano, such compounds may be prepared according to the following route: 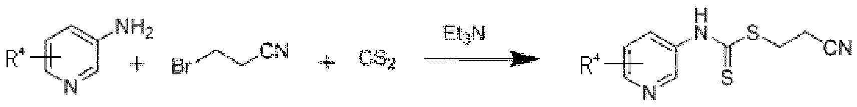 if necessary, the pharmaceutically acceptable salts of the compounds may be prepared according to the routine salt-forming process in the chemical field;
wherein the group R⁴ is a substituent of the group A, which is one or more groups selected from halogen, C₁₋₄alkyl, C₁₋₄alkoxy, phenyl, phenoxy, furyl, oxazinyl, amido and C₁₋₄alkoxycarbonyl; or morpholine ring or benzene ring fused with said pyridyl.

8. A pharmaceutical composition containing a therapeutically effective amount of the compounds of general formula (I) according to any one of Claims 1-5 or the pharmaceutically acceptable salts thereof as an active ingredient, and optionally containing one or more pharmaceutical carrier(s); preferably, of the total weight of said composition, the content of the active ingredient in the pharmaceutical composition is 0.5%-99%, and the content of the pharmaceutical carrier(s) is 1%-99.5%.

9. The pharmaceutical composition according to Claim 8, wherein the composition is formulated into a form for oral administration or parenteral administration, and the form for parenteral administration including the forms for injection administration, topical administration, inhalation administration, rectal administration or implantable administration; the form for oral administration being tablets, capsules, granules or pharmaceutically acceptable liquid preparations; and preferably, the tablets are film-coated, enterosoluble, sustained-release or quantitative-release form.

10. Use of the compounds according to any one of Claims 1-5 or the pharmaceutical compositions according to Claim 8 or Claim 9, for the preparation of tyrosine kinase inhibitors; wherein the tyrosine kinase inhibitors are anti-tumor drugs; preferably, wherein the anti-tumor drugs are used for treating breast cancer, liver cancer, non-small cell lung cancer, gastric cancer, colon cancer, leukaemia or nasal cancer.
